# EUROPEAN PATENT APPLICATION

(11) **EP 4 756 428 A1**
(43) Date of publication of application: **10.06.2026**
(21) Application number: 24217854.9
(22) Date of filing: 05.12.2024
(51) Int. Cl.: G01N 33/50, G01N 33/573, G01N 33/58, C12N 5/00, C07K 7/04

(54) **HYDROGEL-BASED POLYMERIC 3D-SCAFFOLD FOR DETECTING THE VIABILITY OF CELLS**

(71) Applicant: Universität Heidelberg, 69117 Heidelberg (DE)
(72) Inventor: LEAL-EGAÑA, Aldo, 69120 Heidelberg (DE); TAALE, Mohammadreza, 69120 Heidelberg (DE); SCHWEGLER, Niklas, 69120 Heidelberg (DE); THOMAS, Franziska, 69120 Heidelberg (DE); SELHUBER-UNKEL, Christine, 69120 Heidelberg (DE); BLASCO, Eva, 69120 Heidelberg (DE)
(74) Representative: Vossius & Partner Patentanwälte Rechtsanwälte mbB

(57) **Abstract**

The present invention relates to a hydrogel-based polymeric 3D-scaffold, wherein the hydrogel comprises a caspase-cleavable peptide that is functionalized by a fluorescence resonance energy transfer (FRET) pair, wherein the FRET acceptor of the FRET pair stops and the FRET donor of the FRET pair starts or increases emitting fluorescence when the caspase-cleavable peptide is cleaved by a caspase.

## Description

The present invention relates to a hydrogel-based polymeric 3D-scaffold, wherein the hydrogel comprises a caspase-cleavable peptide that is functionalized by a fluorescence resonance energy transfer (FRET) pair, wherein the FRET acceptor of the FRET pair stops and the FRET donor of the FRET pair starts or increases emitting fluorescence when the caspase-cleavable peptide is cleaved by a caspase.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

During the last years, methods used to characterize the activity of cells cultured in three-dimensional (3D) milieus have gained relevance. For example, in the field of cancer research, a series of analytical strategies and/or devices have been developed to quantify the cell viability, with the purpose to validate new treatments against this pathology, including the possibility to discover and screen the activity of new anticancer drugs (or cocktails of them), the establishment of new protocols for radiotherapy or even immunotherapy.

Most of these strategies have been mostly established with the purpose to study the cellular behavior of spheroids/organoids, which nowadays are considered as the gold standard for studying cancer progression.

Regarding the use of spheroids/organoids, in the market there is a series of methods to perform make them in the laboratory, prior analyzing the viability of the cells conforming these spheroids/organoids in presence of several biological, chemical or physical stimuli. Examples of these consumables are the non-adherent plates offered by Facellitate¹, InSphero², or Corning³, to name a few examples. Due to a better capability to resemble cancer pathologies compared to cells cultured on two-dimensional (2D) surfaces, cells cultured as Organoids/Spheroids are likely to be used for this purpose.

In order to study the viability of cells cultured in 3D, there are a series of methods aimed to determine the viability of cells cultured either on 2D and 3D, which are based on the generation of color and/or fluorescence when cells are alive (i.e. Calcein AM⁴, CellTiter-Glo^{®} 3D Cell Viability Assay⁵, etc). Another branch of this business relies on sale of devices aimed to analyze the cell activity in 3D (Incucyte^{®6}, Vi-CELL XR Cell Viability Analyzer' or the CEAxCELLigence RTCA DP Real-Time Cell Analyzer⁸).

Due to the high demand to address cytotoxicological analysis of cells cultured in 3D, several companies have been founded with the aim of supporting the culture of organoids (i.e. faCellitate⁹, InSphero¹⁰), as well as to perform these assays as a service (Ice-Biosc¹¹, Vivitecnia¹², Spherotec¹³).

However, there is an ongoing need for reliable novel systems and methods to determine the viability of cells cultured in 3D *in vitro* (or *ex vivo*).

Accordingly, the present invention relates in a first aspect to a hydrogel-based polymeric 3D-scaffold, wherein the hydrogel comprises a caspase-cleavable peptide that is functionalized by a fluorescence resonance energy transfer (FRET) pair, wherein the FRET acceptor of the FRET pair stops and the FRET donor of the FRET pair starts or increases emitting fluorescence when the caspase-cleavable peptide is cleaved by a caspase.

The term "hydrogel" as used herein refers to a two- or multi-component system consisting of a three-dimensional network of polymer chains and water that fills the space between macromolecules. Depending on the properties of the polymer (or polymers) used, as well as on the nature and density of the network joints, such structures in an equilibrium can contain various amounts of water; typically in the swollen state. The mass fraction of water in a hydrogel is much higher than the mass fraction of a polymer. In practice, to achieve high degrees of swelling, it is common to use synthetic polymers that are water-soluble when in non-cross-linked form. Hydrogels may be synthesized in a number of chemical ways. These include one-step procedures like polymerization and parallel cross-linking of multifunctional monomers, as well as multiple step procedures involving synthesis of polymer molecules having reactive groups and their subsequent cross-linking, possibly also by reacting polymers with suitable cross-linking agents. A polymer engineer can design and synthesize polymer networks with molecular-scale control over structure such as cross-linking density and with tailored properties, such as biodegradation, mechanical strength, and chemical and biological responses to stimuli. A review of the preparation, characterization, and applications of hydrogels can be found, for example, in Ahmed et al. (2015), Journal of Advanced Research, 6(2):105-121.

The hydrogel in accordance with the present invention forms a polymeric 3D-scaffold. A hydrogel-based polymeric 3D-scaffold as referred to herein is a three-dimensional (3D) structure made up at least in part by polymers that build the hydrogel.

As will be further explained herein below, the hydrogel-based polymeric 3D-scaffold is capable of entrapping cells, in particular viable cells. The purpose "for entrapping cells" defines that the 3D-scaffold has dimensions which are suitable to incorporate one or more cells or cell types (i.e. different populations of cells) and preferably also for growing the cell(s) within the 3D-scaffold. The purpose "for entrapping cells" defines that the 3D-scaffold has suitable chemical composition for entrapping cells, in particular that the material of the 3D-scaffold is non-toxic for the cells.

It is to be understood that the hydrogel-based polymeric 3D-scaffold of the invention is capable of indicating the death, preferably the apoptosis of cells being entrapped within the 3D-scaffold. The death, preferably the apoptosis of cells is indicated by the stop of the fluorescence of the FRET acceptor and the start or increase of the fluorescence of the FRET donor. This stop of the fluorescence of the FRET acceptor and the start or increase of the fluorescence of the FRET donor also allows to determine within the 3D-scaffold the three-dimensional localization of the dead cells.

In case a FRET pair is in right orientation and proximity - e.g. in the case of a caspase-cleavable peptide that is functionalized by a FRET pair - the FRET acceptor emits fluorescence while the fluorescence of the FRET donor is reduced, if the system is excited at the emission wavelength of the FRET donor. This is because the FRET donor transfers energy to the FRET acceptor. If the FRET donor and acceptor are separated - e.g. because a caspase cleaves the caspase-cleavable peptide that is functionalized by a FRET pair - only the fluorescence of the FRET donor remains but no longer fluorescence of the FRET acceptor can be detected. This because when the caspase-cleavable peptide is cleaved the FRET donor and the FRET acceptor are no longer in close proximity; the FRET donor can no longer transfer energy to the FRET acceptor. Hence, the fluorescence of the FRET acceptor is turned off but in turn the fluorescence of the FRET donor is generally turned on/increased, if the system is excited at the emission wavelength of the FRET donor.

Fluorescence resonance energy transfer (FRET) is generally a mechanism describing energy transfer between two light-sensitive molecules. A donor chromophore, initially in its electronic excited state, may transfer energy to an acceptor chromophore through non-radiative dipole-dipole coupling. The efficiency of this energy transfer is inversely proportional to the sixth power of the distance between donor and acceptor, making FRET extremely sensitive to small changes in distance. Methods to measure FRET efficiency are well-known and include fluorescence microscopy, such as fluorescence confocal laser scanning microscopy.

Caspases (cysteine-aspartic proteases, cysteine aspartases or cysteine-dependent aspartate-directed proteases) are a family of protease enzymes playing essential roles in programmed cell death. They are named caspases due to their specific cysteine protease activity - a cysteine in its active site nucleophilically attacks and cleaves a target protein only after an aspartic acid residue. There are 12 confirmed caspases in humans and 10 in mice, carrying out a variety of cellular functions (Saleh et al. (2004) Nature. 429 (6987): 75-9.). Caspases preferentially cleave certain amino acid motifs and these motifs can be used to design caspase-cleavable peptides (McStay et al. Cell Death & Differentiation volume 15, pages 322-331 (2008)). Preferred examples of caspases and caspase-cleavable peptides will be provided herein below.

As shown in the appended examples, the hydrogel-based polymeric 3D-scaffold can be used to entrap cultured cells of a cell line (Example 4), a mixture of different cell types (Example 5) as well as primary cells that were isolated from a subject (Example 6). In all three cases an anticancer drug, such as cisplatin can be used to induce the cell death of the entrapped cells. The death of the entrapped cells is then indicated by the stop of the fluorescence of the FRET acceptor and the start or increase of the fluorescence of the FRET donor. This can be determined by visual inspection, such by confocal microscopy. The the total mortality of the entrapped cells can thereby be determined.

In accordance with a preferred embodiment of the first aspect the caspase is caspase 3, 6 or 7 and is preferably caspase 3.

Caspase 3, 6 and 7 are the so-called executioner caspases. The executioner caspases are cleaved and activated by initiator caspases (such as Caspase 9). The active executioner caspases then degrade cellular components for apoptosis (McStay et al. Cell Death & Differentiation volume 15, pages 322-331 (2008)).

Caspase-3 cleaves the peptide DEVD (SEQ ID NO: 2) most efficiently but cleaves other substrates as well. Similarly, caspase-6 cleaves the peptide VEID (SEQ ID NO: 3) most efficiently but also demonstrates efficient cleavage of the peptides IETD (SEQ ID NO: 4) and LEHD (SEQ ID NO: 5). Caspase-7 has a similar profile to caspase-3, cleaving DEVD most efficiently (McStay et al. Cell Death & Differentiation volume 15, pages 322-331 (2008)).

Caspase 3 is most preferred because it is the most promiscuous and efficient caspase among the executioner caspases. Its activity is the most prevalent after engagement of the mitochondrial pathway of apoptosis.

In accordance with a further preferred embodiment of the first aspect, the caspase-cleavable peptide comprises the amino acids DXXD/G, wherein D is Asp, X is any amino acid, and G is Gly (SEQ ID NO: 1), and preferably comprises the amino acids Asp-Glu-Val-Asp (SEQ ID NO: 2).

The amino acid motif DXXD/G, wherein D is Asp, X is any amino acid, and G is Gly (SEQ ID NO: 1) defines peptides that are preferably and selectively cleaved by Caspase 3 while Asp-Glu-Val-Asp (SEQ ID NO: 2) is the peptide being most preferably and most selectively cleaved by Caspase 3.

In accordance with another preferred embodiment of the first aspect, the donor of the FRET pair is linked to the most C-terminal amino acid of the caspase-cleavable peptide and the acceptor of the FRET pair is linked to the most N-terminal amino acid of the caspase-cleavable peptide or *vice vera.*

The linkage is preferably a covalent linkage. Means and methods for linking FRET donor and FRET acceptor to peptides are art-established (see, e.g., Shah et al (2016), Methods Appl Fluoresc; 4(4):047002 and Bajar et a. (2016), Sensors (Basel). 2016 Sep; 16(9): 1488.).

As discussed above, when a FRET pair is at the ends of the short caspase-cleavable peptide the FRET donor can transfer energy to the FRET acceptor, because they are in close proximity.

In accordance with a preferred embodiment of the first aspect, the caspase-cleavable peptide comprises the amino acids Xaa-Asp-Glu-Val-Asp-Xaa (SEQ ID NO: 6), wherein Xaa is an amino acid, preferably Asp or Lys.

The peptide Xaa-Asp-Glu-Val-Asp-Xaa (SEQ ID NO: 6) comprises the Casp 3 cleavable peptide Asp-Glu-Val-Asp (SEQ ID NO: 2) and in addition at the two termini any amino acid and preferably Asp or Lys. The two further amino acids and in particular the side chains thereof can be used to covalently link the FRET pair to the peptide without interfering with the Casp 3 cleavable peptide *per se.* Asp or Lys are preferred because they comprise a free functional group - a -COOH (carboxy) and a -NH₂ (amino) group, respectively - in their side chain and the FRET pair members can be easily attached via this free functional -NH₂ (amino) group.

Any caspase cleavable peptide as described herein can comprise in addition any amino acid and preferably Asp or Lys at its termini, just as shown in SEQ ID NO: 6 for the Casp 3 cleavable peptide of SEQ ID NO: 2.

In accordance with a preferred embodiment of the first aspect,
(i) the acceptor of the FRET pair is DABCYL (4-((4-(dimethylamino)phenyl)azo)benzoic acid) and the donor of the FRET pair is EDANS (5-((2-Aminoethyl)amino)naphthalene-1-sulfonic acid),
(ii) the acceptor of the FRET pair is TAMRA (carboxytetramethylrhodamin) and the donor of the FRET pair is FAM (fluoresceine amidite)) or FITC (fluorescein isothiocyanate), or
(iii) the acceptor of the FRET pair is Cy5 and the donor of the FRET pair is Cy3,
and wherein the caspase-cleavable peptide preferably is Asp(EDANS)-Asp-Glu-Val-Asp-Lys(DABCYL) (SEQ ID NO: 7).

While the FRET pairs according to items (i) to (iii) are preferred the FRET pair is not particularly limited and a number of suitable FRET pairs are known in the prior art. Cy3 and Cy5 are cyanine dyes (tetramethylindo(di)-carbocyanines).

Item (i) is preferred and Asp(EDANS)-Asp-Glu-Val-Asp-Lys(DABCYL) (SEQ ID NO: 7) is most preferred because this FRET pair / FRET pair caspase-cleavable peptide construct is used in the appended examples.

In accordance with another preferred embodiment of the first aspect, the 3D-scaffold comprises or consists of gelatin and alginate, or chitosan and gelatin, or silk and polyethylene glycol and gelatin, or alginate and fibronectin, or alginate and Matrigel, or chitosan and Matrigel, or a 3D printer ink.

3D-scaffolds made of these polymers are particularly advantageous for entrapping cells. Gelatin and alginate are most preferred since they are used in the examples.

Matrigel is the trade name for the solubilized basement membrane matrix secreted by Engelbreth-Holm-Swarm (EHS) mouse sarcoma cells. Matrigel resembles the laminin/collagen IV-rich basement membrane extracellular environment found in many tissues and is used by cell biologists as a substrate (basement membrane matrix) for culturing cells.

Preferred examples of 3D printer inks are made of PEGDA/PETA (polyethylene glycol) diacrylate and pentaerithritol tetraacrylate), poly(methyl methacrylate), polyacrilamide, or GelMa (gelatinemethacryloyl).

In accordance with a preferred embodiment of the first aspect, the 3D-scaffold is a microcapsule, preferably having a diameter of about 100 µm to about 1200 µm, most preferably of about 100 µm to about 600 µm.

While the shape of 3D-scaffold is flexible microcapsules have a spheric shape. The diameter of a microcapsule is preferably about 100 µm to about 1200 µm, most preferably about 100 µm to about 600 µm.

These diameter ranges are particularly well suitable for entrapping cells and for determining their death, preferably the apoptosis of cells being entrapped within the microcapsules.

Accordingly also the 3D-scaffold as described herein preferably has a width, length and breadth in the range of about 100 µm to about 1200 µm, most preferably about 100 µm to about 600 µm.

The term "about" as used herein means with increasing preference ±20%, ±10% and ±5% of the respective value being characterized by the term "about". The exact values without the term "about" are most preferred.

In accordance with a further preferred embodiment of the first aspect, the hydrogel further comprises one or more of
(a) a peptide mediating cell-matrix attachment,
(b) a peptide comprising extracellular matrix (ECM) protein-binding domain, and
(c) a peptide comprising a domain being recognized by a metalloproteinase,
(d) a peptide comprising a domain for a growth factor,
(e) a peptide comprising a domain being recognized by Lysyl oxidase,
wherein the peptide(s) of one or more of (a) to (e) are preferably covalently attached to the hydrogel.

In accordance with a more preferred embodiment of the first aspect,
(a) the peptide of (a) comprises or consists of a linear or cyclic RGD peptide, GFOGERGVEGPOGPA, LPGER, GLPGER, GXOGER, GFOGE, or CDPGYIGSR (SEQ ID NOs 8 to 13),
(b) the peptide of (b) comprises or consists of IQNNKFEYKEDTIKE, TKKTLRT or LRELHLNNN (SEQ ID NOs 14 to 16),
(c) the peptide of (c) comprises or consists of Gly-Leu, Gly-Ile, ALRRLLGLFG, ALRRLLSLF, XXX_{Hy}, L/IXXX_{Hy}, PVPQGS (SEQ ID NOs 17 to 23), wherein X can be any amino acid and X_{HY} is a hydrophobic amino acid,
(d) the peptide of (d) comprises or consists of MTAASMGPVRVAFVVLLALCSRPAVG, DYLAGFKAHGKKYR and CTCCC (SEQ ID NOs 24 to 26); and/or
(e) the peptide of (e) is rich in Lysyl residues.

The functionalization of hydrogels with peptides according to the above items (a) to (e) of the preferred and more preferred embodiment is known in the art. For example, RGD-peptide modified alginate is described in Sandvig et al. (2015), J Biomed Mater Res A; 103(3):896-906 and for a review on functionalized hydrogels reference is made to Enayati et al. (2024), Advances in Colloid and Interface Science, 331:103232. Also click chemistries can be used for the functionalization of hydrogels; Hui et al. (2021), Mol. Syst. Des. Eng., 6:670-707.

The hydrophobic amino acid is preferably selected from glycine (Gly), alanine (Ala), valine (Val), leucine (Leu), isoleucine (Ile), proline (Pro), phenylalanine (Phe), methionine (Met), and tryptophan (Trp).

The peptides of (a) and (b) can be used to attach the cells to the 3D-scaffold.

Metalloproteases are secreted proteins that have the capacity to cleave extracellular matrix proteins (ECM proteins). Because metalloproteinases can degrade ECM molecules, their main function has been presumed to be remodeling of the ECM. Hence, by the peptide of (c) the metalloproteases can be directed to the 3D-scaffold where they might remodel the 3D-scaffold (noting that the 3D-scaffold might comprise components of the ECM, such a fibronectin) and the ECM of the entrapped cells.

A growth factor is a naturally occurring substance capable of stimulating cell proliferation, and occasionally cellular differentiation. Hence, by the peptide of (d) the cell proliferation within the 3D-scaffold can be controlled.

Lysyl oxidase is an extracellular copper-dependent enzyme that catalyzes formation of aldehydes from lysine residues in collagen and elastin precursors. Hence, by the peptide of (e) Lysyl oxidase can be directed to the 3D-scaffold where they might remodel the lysine residues of the 3D-scaffold and/or the cells.

In accordance with a further preferred embodiment of the first aspect the 3D-scaffold comprises viable cells, preferably viable malignant cells, most preferably viable tumor cells or viable cancer cells.

As discussed herein above, the hydrogel-based polymeric 3D-scaffold is capable of entrapping cells, in particular viable cells. In accordance with the above preferred embodiment the viable cells are actually entrapped within the hydrogel-based polymeric 3D-scaffold.

Viable cells have the ability of having metabolic activity, growth, and proliferate. Malignant cells can invade and then grow in an uncontrolled way in nearby tissues and spread to other parts of the body through the blood and lymph system. After invading new colonized tissues, to form secondary tumors, tumor cells divide continually, forming solid tumors.

The present invention relates in a second aspect to a platform for analyzing the viability of cells, wherein the platform comprises at least one unit that comprises
(a) a chamber that comprises one or more, preferably one of the 3D-scaffolds of the first aspect and preferably one that comprises viable cells, preferably viable malignant cells, most preferably viable tumor cells or viable cancer cells
(b) an inlet microchannel that is in fluid connection with the at least one chamber and through which a fluid with a test compound can be introduced into the chamber, and
(c) optionally an outlet microchannel that is in fluid connection with the at least one chamber and through which the fluid with the test compound can leave the chamber.

The definitions and preferred embodiments as described herein above for the first aspect apply *mutatis mutandis* to the second aspect of the invention as far as being amenable therewith and *vice versa.*

Hence, the 3D-scaffolds of the first aspect may form a part of a platform for analyzing the viability of cells. Within each unit of this platform one or more, preferably one of the 3D-scaffolds of the first aspect are within a chamber. In the chamber the viable cells can be cultured, and it can be detected via the above-described FRET pair whether the cells being entrapped within the scaffold die. The chamber is connected to an inlet microchannel through which a fluid with a test compound can be introduced into the chamber. The fluid is preferably a maintenance or proliferation medium for the cells being entrapped with the 3D-scaffod. The test compound is a compound for which it can be determined whether it has an influence on the viability of the cells. For example, such a test compound can be a drug that is not affecting the proliferation of the cells, but some metabolic/physiological mechanism, such as the drug Paclitaxel, which disrupt the cytoskeleton, by binding tubulin. This is preferably done by comparison to a negative control in another unit of the cells, wherein unit comprises all the same materials with the exception of the test compound. The chamber is optionally connected to an outlet microchannel through which a fluid with a test compound can leave the chamber. The inlet and outlet microchannels can be used to generate a fluid flow, to exchange the fluid, medium or the test compound.

In accordance with a preferred embodiment of the second aspect the system comprises with increasing preference at least 10, at least 50 and at least 100 units, preferably in parallel.

This system with these numbers of units allows to test servals kinds of 3D-scaffods, types of cells, entire fluids or only media or test compounds in parallel even in a high-throughput manner. The FRET fluorescence and hence the viability of the cells can be red out in each of the chambers separately, for example, by a fluorescent microscope being coupled to a CCD camera.

In accordance with another preferred embodiment of the second aspect, the chamber(s) have a width of about 300-900 µm and a height of about 200-900 µm, and/or
the channel(s) have a width of about 50 µm and a height of about 20-50 µm, and wherein the channel(s) optionally have and inlet or outlet having a width of about 200 µm and a height of about 20 µm.

The chamber preferably also has a diameter of 300-900 µm. The length of the channel(s) can be selected as needed in order optimize fluid provision and, if present, removal.

In accordance with a preferred embodiment of the second aspect, the unit(s) is/are bound to a surface, preferably a glass or plastic surface.

Many different plastic types have been used over the years for cell culture and cell-based assays. Preferred plastic surfaces are made of one or more of is polystyrene (PS), polyethylene terephthalate (PET), high- and low-density polyethylene (PE), polyvinyl chloride (PVC), and polypropylene (PP), wherein polystyrene (PS) is most preferred.

In accordance with a preferred embodiment of the second aspect, the chamber(s) is/are made of silicone and/or the channel(s) is/are made of a poly(ethylene glycol) diacrylate (PEGDA)/ pentaerithritol tetraacrylate (PETA)-based hydrogel or polyacrylamide (PAA).

The present invention relates in a third aspect to a method for testing whether a test compound or a test condition induces the death, preferably apoptosis of cells comprising (a) introducing a test compound into the 3D-scaffold of the first aspect that comprises viable cells, or contacting a fluid with a test compound with the 3D-scaffold of the first aspect that comprises viable cells, or contacting a fluid with a test compound with the system of the second aspect that comprises viable cells, so that the test compound is introduced into the chamber, or applying a test condition to the 3D-scaffold of the first aspect that comprises viable cells, or applying a test condition to the system of the second aspect that comprises viable cells; and (b) monitoring the fluorescence of the FRET pair, wherein a decreased or no fluorescence of the FRET acceptor of the FRET pair and/or increased or the onset of fluorescence of the FRET acceptor of the FRET pair upon the contact of the test compound with the cells in the 3D-scaffold(s) or the appliance of the test condition to the cells in the 3D-scaffold(s) indicates that the compound induces the death, preferably apoptosis of the cells.

The definitions and preferred embodiments as described herein above for the first and second aspect apply *mutatis mutandis* to the third aspect of the invention as far as being amenable therewith and *vice vera.*

The method of the third aspect of the invention employs the 3D-scaffold of the first aspect or the system of the second aspect for testing whether a test compound or a test condition induces the death, preferably apoptosis of the cells being entrapped in the 3D-scaffold.

This can be done in accordance with step (a) by first (a1) introducing a test compound into the 3D-scaffold of the first aspect that comprises viable cells, (a2) contacting a fluid with a test compound with the 3D-scaffold of the first aspect that comprises viable cells, (a3) contacting a fluid with a test compound with the system of the second aspect that comprises viable cells, so that the test compound is introduced into the chamber, (a4) applying a test condition to the 3D-scaffold of the first aspect that comprises viable cells, or (a5) applying a test condition to the system of the second aspect that comprises viable cells. After the test compound or test condition has been applied in step (a) in step (b) the fluorescence of the FRET pair is monitored (wherein the system is excited at the emission wavelength of the FRET donor). A decreased or no fluorescence of the FRET acceptor of the FRET pair upon the contact of the test compound with the cells in the 3D-scaffold(s) or the appliance of the test condition to the cells in the 3D-scaffold(s) and excitation at the emission wavelength of the FRET donor indicates that the compound induces the death, preferably apoptosis of the cells.

This is because when the cells undergo cell death, preferably apoptosis, caspase becomes activated, the caspase cleavable peptide is cleaved by the active caspase and the FRET pair is no longer in close proximity. The FRET donor can longer transfer energy to the FRET acceptor upon excitation at the emission wavelength of the FRET donor. The consequence is that the fluorescence of the FRET acceptor is reduced or stopped and the fluorescence of the FRET donor starts or increases (depending on how many cells die).

In accordance with a preferred embodiment of the third aspect the method is an *in vitro* or *ex vivo* method.

An *in vitro* or *ex vivo* method is performed outside a mammalian human or animal organism as opposed to an *in vivo* method.

In accordance with a preferred embodiment of the third aspect the test compound is a small molecule, a drug, a toxic compound, an antibody, an antibody-mimetic, a cytokine, or a chemokine; and/or the test condition is radiation, pressure, heating, cooling, or a vacuum.

The "small molecule" as used herein is preferably an organic molecule. Organic molecules relate or belong to the class of chemical compounds that contain both, hydrogen and carbon. The organic molecule is preferably an aromatic molecule and more preferably a heteroaromatic molecule. In a heteroaromatic molecule at least one of the atoms in the aromatic ring is an atom other than carbon, e.g. N, S, or O. For all above-described organic molecules the molecular weight is preferably in the range of 200 Da to 1500 Da and more preferably in the range of 300 Da to 1000 Da.

Alternatively, the "small molecule" in accordance with the present invention may be an inorganic compound. Inorganic compounds can be derived from mineral sources and include all compounds without carbon atoms (except carbon dioxide, carbon monoxide; elementary carbon and carbonates). Preferably, the small molecule has a molecular weight of less than about 2000 Da, or less than about 1000 Da such as less than about 500 Da, and even more preferably less than about Da amu. Non-limiting but preferred examples of a small molecule that can be use to treat cancer are just high concentrations or Bromine (Br₂), or Lithium as well as radioactive atoms, such as enriched Uranium.

A drug is a naturally or artificially made chemical that is used as a medicine.

The toxic compound is preferably a small organic compound, more preferably a toxic compound selected from the group consisting of calicheamicin, maytansinoid, neocarzinostatin, esperamicin, dynemicin, kedarcidin, maduropeptin, doxorubicin, daunorubicin, and auristatin. A proteinaceous toxic compound is preferably Ricin-A chain, modeccin, truncated Pseudomonas exotoxin A, diphtheria toxin and recombinant gelonin.

The term "antibody" as used in accordance with the present invention comprises, for example, polyclonal or monoclonal antibodies. Furthermore, also derivatives or fragments thereof, which still retain the binding specificity to the target are comprised in the term "antibody". Antibody fragments or derivatives comprise, inter alia, nanobodies (or single-domain antibody (sdAb)), Fab or Fab' fragments, Fd, F(ab')2, Fv or scFv fragments, single domain VH or V-like domains, such as VhH or V-NAR-domains, as well as multimeric formats such as minibodies, diabodies, tribodies or triplebodies, tetrabodies or chemically conjugated Fab'-multimers (see, for example, Harlow and Lane "Antibodies, A Laboratory Manual", Cold Spring Harbor Laboratory Press, 198; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126).

As used herein, the term "antibody mimetics" refers to compounds which, like antibodies, can specifically bind antigens, but which are not structurally related to antibodies. Antibody mimetics are usually artificial peptides or proteins with a molar mass of about 3 to 20 kDa. For example, an antibody mimetic may be selected from the group consisting of affibodies, adnectins, anticalins, DARPins, avimers, nanofitins, affilins, Kunitz domain peptides, Fynomers, trispecific binding molecules and prododies. These polypeptides are well known in the art.

The cytokine is preferably selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-ligand, TGF-beta, IL-1alpha and IL-1beta. As it is well-known in the art, cytokines may favour a pro-inflammatory or an anti-inflammatory response of the immune system.

The chemokine is preferably selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, eotaxin, Eotaxin-2, I-309, MPIF-1, 6Ckine, CTACK, MEC, lymphotactin and fractalkine. The major role of chemokines is to act as a chemoattractant to guide the migration of cells. Cells that are attracted by chemokines follow a signal of increasing chemokine concentration towards the source of the chemokine.

Radiation, pressure, heating, cooling, and a vacuum are all conditions that can influence the viability of cells.

In accordance with a preferred embodiment of the third aspect the cells are cultured in the presence of the test compound for a time wherein the cells can proliferate.

Cell proliferation is the process by which a cell grows and divides to produce two daughter cells. Means and methods, in particular culture conditions and media for cell growth are known in the art.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.
**Figure 1****:** MALDI-TOF MS spectrum of pure CCP1-nA peptide (reflector negative mode 200 3000, main peak at 1216.5 m/z corresponds to [M-H]-, peak at 1084.5 m/z is caused by dye fragmentation of the peptide during laser exposure).
**Figure 2****:** Analytical HPLC trace of pure CCP1-nA peptide (20-50% B in A, 220 nm).
**Figure 3****:** Analytical HPLC trace of pure CCP1-nA peptide (20-50% B in A, 280 nm).
**Figure 4****:** MALDI-TOF MS spectrum of pure CCP1 peptide (reflector negative mode 400 4000, main peak at 1270.4 m/z corresponds to [M-H]-, peak at 1138.3 m/z is caused by dye fragmentation of the peptide during laser exposure).
**Figure 5****:** Analytical HPLC trace of pure CCP1 peptide (15-50% B in A, 220 nm).
**Figure 6****:** Analytical HPLC trace of pure CCP1 peptide (15-50% B in A, 280 nm).

The examples illustrate the invention.

### Example 1 - Peptide synthesis

Peptides were synthesized by solid phase synthesis at a 50 µmol scale on a Fmoc-Rink-Amide MBHA polystyrene resin with a loading density of 0.67 mmol/g. For coupling steps, Fmoc protected amino acid (100 µmol, 2 eq), PyBOP (52 mg, 100 µmol, 2 eq), and DIPEA (34 µL, 200 µmol, 4 eq) were dissolved in 1 mL DMF and added to resin (50 µmol, 1 eq). Mixture was equilibrated for 45 min, then resin washed with DMF five times. For Fmoc deprotection steps, 4 mL of 20vol% piperidine solution in DMF were added to resin, equilibrated for 5 min. Procedure was repeated once. Resin was washed with DMF five times. For final cleavage of the peptides from the resin and removal of acid-labile side chain protecting groups, dried resin was combined with 5 mL of a TFA, water and TIPS mixture (95:2.5:2.5 volume ratio) and equilibrated for 3 h. Supernatant was extracted and resin washed with 2x2 mL TFA. TFA was evaporated under nitrogen stream from combined liquids (final volume ^{~}2 mL). Peptide was precipitated by adding 15 mL ice cold diethyl ether to remaining liquids. Precipitate was extracted by centrifugation (8 min, 10,000 rpm) and decantation. Pellet was washed with 10 mL ice cold diethyl ether and dried in vacuo.

### Example 2 - Peptide purification and analysis

Peptides were purified by reversed-phase HPLC on a VDS optilab VDSpher PUR 100 C18-SE (250 mm x 10 mm, 100 Å, 5 µm) column with a flow rate of 3 mL/min and at a temperature of 50 °C. AJasco device with a PU-4180 pump, a CO-4060 column thermostat and a UV-4070 detector was used. Analytical HPLC was performed with a Hitachi Primaide device consisting of a 1110 Pump, a 1210 auto sampler, 1310 column oven and a 1430 diode array detector. A VDS optilab VDSpher PUR 100 C18-SE (250 mm x 4.6 mm, 100 Å, 5 µm) column with a flow rate of 1 mL/min and a temperature of 50 °C was used. A binary solvent system with buffer A (ultrapure water with 0.1% TFA) and buffer B (acetonitrile with 0.1% TFA) was used. Gradient programs are indicated in the respective figures with the nomenclature X-Y% B in A, whereas X refers to the ratio of buffer B in buffer A during initial equilibration (5 min), and Y refers to the final ratio of B in A after 30 min of linear gradient. During minute 35 to 50 of the HPLC programs, the column is washed and re-equilibrated to the starting ratio. Matrix-assisted laser desorption ionization time-of-flight mass spectrometry (MALDI-TOF MS) was performed on a Bruker Autoflex Speed device. Samples were prepared by mixing 1 µL of sample with 1 µL of matrix solution (20 mg mL⁻¹ 2,5-dihydroxybenzoic acid in 30% B in A). Measuring parameters are indicated in the respective figures.

### Example 3 - Peptide specifications

| | | |
|---|---|---|
| CCP1-nA | Sequence Mass Purification Analysis | H-Asp(EDANS)-Asp-Glu-Val-Asp-Lys(DABCYL)-NH₂ 1218.3 g/mol Semipreparative HPLC, 15-50% B in A, retention time: 20 min Analytical HPLC, 20-50% B in A, retention time: 17 min MALDI-TOF MS, calc. [M-H]⁻ 1216.5 m/z, found [M-H]⁻ 1216.5 m/z |
| CCP1 | Sequence Mass Purification Analysis | Acryloyl-Asp(EDANS)-Asp-Glu-Val-Asp-Lys(DABCYL)-NH₂ 1272.4 g/mol Semipreparative HPLC, 25-35% B in A, retention time: 15 min Analytical HPLC, 20-50% B in A, retention time: 22 min MALDI-TOF MS, calc. [M-H]⁻ 1270.5 m/z, found [M-H]⁻ 1270.4 m/z |

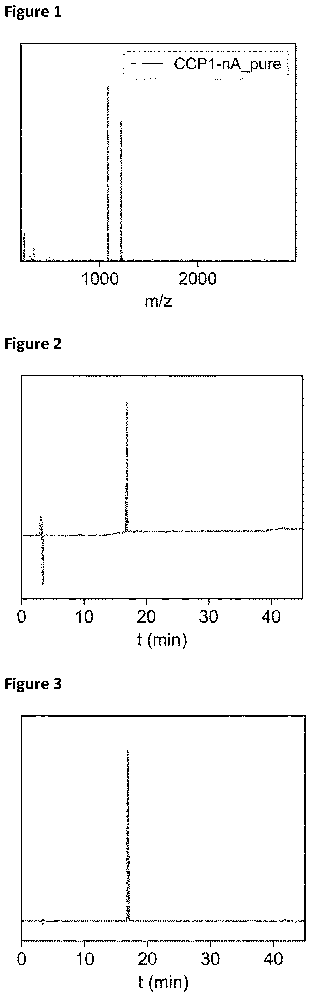

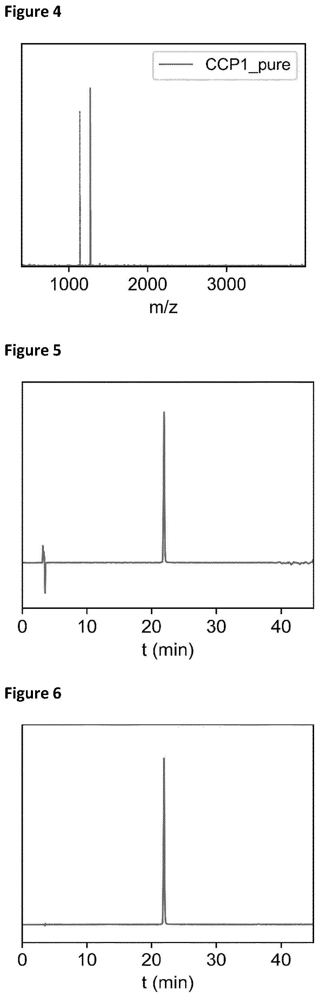

MALDI-TOF MS spectrum of pure CCP1-nA peptide and pure CCP1 peptide are shown in Figures 1 and 4. Analytical HPLC trace of pure CCP1-nA peptide are shown in Figures 2 and 3 (20-50% B in A, 220 nm and 280) while analytical HPLC trace of pure CCP1 peptide are shown in Figures 4 and 5 (20-50% B in A, 220 nm and 280).

### Example 4 - MCF7 breast cancer cells entrapped in microcapsules made of 1.0% w/v alginate-gelatin (ratio 1:1)

MCF7 breast cancer cells are entrapped in microcapsules made of 1.0% w/v alginate-gelatin (ratio 1:1). MCF7 breast cancer cells are entrapped in microcapsules made of 1.0% w/v alginate-gelatin (ratio 1:1). In these polymer blend, the alginate has been functionalized with the FRET sensible peptide domains, additionally containing the cell-adhesive peptide RGD. These microcapsules are placed in the 3D printed structure. These 3D structures, containing the microcapsules, will be then immersed in cell culture media. After this, a solution containing the anticancer drug cisplatin will be supplied in concentrations between 0 (control) and 100 µM. After exposing the encapsulated cells to the drug (in the afore-mentioned concentrations), the capsules, containing embedded MCF7 cells will be imaged with a confocal microscope. The three-dimensional reconstruction of the images will show the number of spots where fluorescence, as a product of FRET, is observed. This enables to determine the total mortality of cancer cells found inside the microcapsules. Additionally, the three-dimensional reconstruction of the images will indicate the positioning of the cells within the three-dimensional microcapsules, enabling to determine the localization of dead cells in depth.

### Example 5 - MCF7 breast cancer cells, primary human fibroblasts and endothelial cells (for example, the cell line EA.hy926) co-immobilized in microcapsules made of 1.0% w/v alginate-gelatin (ratio 1:1)

MCF7 breast cancer cells, primary human fibroblasts and endothelial cells (for example, the cell line EA.hy926) are co-immobilized in microcapsules made of 1.0% w/v alginate-gelatin (ratio 1:1). In these polymer blend, the alginate has been functionalized with the FRET sensible peptide domains, additionally containing the cell-adhesive peptide RGD. These microcapsules are placed in the 3D printed structure. These 3D structures, containing the microcapsules, will be then immersed in cell culture media. After this, a solution containing the anticancer drug cisplatin will be supplied in concentrations between 0 (control) and 100 µM. After exposing the encapsulated cells to the drug (in the afore-mentioned concentrations), the capsules, containing embedded MCF7, human fibroblasts and endothelial cells will be imaged with a confocal microscope. The three-dimensional reconstruction of the images will show the number of spots where fluorescence, as a product of FRET, is observed. This enables to determine the total mortality of cells located inside the microcapsules, information which is used to determine the secondary effects the drugs (non-specific cytotoxicity), after exposing cancer and non-cancer cells to this drug. Additionally, the three-dimensional reconstruction of the images will indicate the positioning of the cancer and non-cancer cells within the three-dimensional microcapsules, enabling to determine if cancer and non-cancer cells interact when they are exposed to a chemotherapeutical drug.

### Example 6 - Primary breast cancer cells immobilized in microcapsules made of 0.8% w/v alginate-gelatin (ratio 1:1).

Primary breast cancer cells, extracted from human patients are immobilized in microcapsules made of 0.8% w/v alginate-gelatin (ratio 1:1). In these polymer blend, the alginate has been functionalized with the FRET sensible peptide domains, additionally containing the cell-adhesive peptide RGD. These microcapsules are placed in the 3D printed structure. These 3D structures, containing the microcapsules, will be then immersed in cell culture media. After this, a solution containing the anticancer drug cisplatin will be supplied in concentrations between 0 (control) and 100 µM. After exposing the encapsulated cells to the drug (in the afore-mentioned concentrations), the capsules, containing embedded primary human cancer cells will be imaged with a confocal microscope. The three-dimensional reconstruction of the images will show the number of spots where fluorescence, as a product of FRET, is observed. This enables to determine the total mortality of primary cancer cells located inside the microcapsules. Additionally, the three-dimensional reconstruction of the images will indicate the positioning of the cells within the three-dimensional microcapsules, enabling to determine the localization of dead cells in depth.

### References

1. https://facellitate.com/modelinq-infectious-diseases-in-3d-human-orqanoids/
2. https://shop.insphero.com/collections/akura%E2%84%A2-3d-spheroid-microplate-solutions?qad source=1&qclid=EAlalQobChMlveD7zpuhhQM\/iWVBAhOBeqRTEAAYAYAAEqlE7fD BwE
3. https://www.corninq.com/au/en/products/life-sciences/products/surfaces/ultra-low-attachment-surface.html
4. https://www.thermofisher.com/order/cataloq/product/C3099?SID=srch-srp-C3099
5. https://www.promeqa.de/en/products/cell-health-assavs/cell-viabilitv-and-cytotoxicitv-assavs/celltiter-qlo-3d-cell-viabilitv-assay/?catNum=G9681
6. https://www.sartorius.com/en/applications/life-science-research/live-cell-assays/spheroid-qrowth
7. https://www.bostonind.com/beckman-vi-cell-xr-cell-viability-analyzer?utm source=merchant shoppinq&utm medium=europe&utm source=qooqle&utm medium =cpc&utm campaiqn=lndividual - Europe - Analyzers&qad source=1&qclid=EAlalQobChMl-si2saShhQMV8oVoCR2P9QxWEAYYASABEqlp8PD BwE
8. https://www.aqilent.com/en/product/cell-analysis/real-time-cell-analvsis
9. https://facellitate.com/
10. https://insphero.com/
11. https://en.ice-biosci.com/
12. https://www.vivotecnia.com/
13. https://www.spherotech.com/

## Claims

1. A hydrogel-based polymeric 3D-scaffold, wherein the hydrogel comprises a caspase-cleavable peptide that is functionalized by a fluorescence resonance energy transfer (FRET) pair, wherein the FRET acceptor of the FRET pair stops and the FRET donor of the FRET pair starts or increases emitting fluorescence when the caspase-cleavable peptide is cleaved by a caspase.

2. The 3D-scaffold of claim 1, wherein the caspase is caspase 3, 6 or 7 and is preferably caspase 3.

3. The 3D-scaffold of claim 1 or 2, wherein the caspase-cleavable peptide comprises the amino acids DXXD/G, wherein D is Asp, X is any amino acid, and G is Gly (SEQ ID NO: 1), and preferably comprises the amino acids Asp-Glu-Val-Asp (SEQ ID NO: 2).

4. The 3D-scaffold of any one of claims 1 to 3, wherein the donor of the FRET pair is linked to the most C-terminal amino acid of the caspase-cleavable peptide and the acceptor of the FRET pair is linked to the most N-terminal amino acid of the caspase-cleavable peptide or *vice vera.*

5. The 3D-scaffold of any one of claims 1 to 4, wherein the caspase-cleavable peptide comprises the amino acids Xaa-Asp-Glu-Val-Asp-Xaa (SEQ ID NO: 6), wherein Xaa is an amino acid, preferably Asp or Lys.

6. The 3D-scaffold of any one of claims 1 to 5, wherein
(i) the acceptor of the FRET pair is DABCYL (4-((4-(dimethylamino)phenyl)azo)benzoic acid) and the donor of the FRET pair is EDANS (5-((2-Aminoethyl)amino)naphthalene-1-sulfonic acid),
(ii) the acceptor of the FRET pair is TAMRA and the donor of the FRET pair is FAM or FITC, or
(iii) the acceptor of the FRET pair is Cy5 and the donor of the FRET pair is Cy3,
and wherein the caspase-cleavable peptide preferably is Asp(EDANS)-Asp-Glu-Val-Asp-Lys(DABCYL) (SEQ ID NO: 7).

7. The 3D-scaffold of any one of claims 1 to 6, wherein the 3D-scaffold comprises or consists of gelatin and alginate, or chitosan and gelatin, or silk and polyethylene glycol and gelatin, or alginate and fibronectin, or alginate and Matrigel, or chitosan and Matrigel, or a 3D printer ink.

8. The 3D-scaffold of any one of claims 1 to 7, wherein the 3D-scaffold is a microcapsule, preferably having a diameter of about 100 µm to about 1200 µm, most preferably of about 100 µm to about 600 µm.

9. The 3D-scaffold of any one of claims 1 to 8, wherein the hydrogel further comprises one or more of
(a) a peptide mediating cell-matrix attachment,
(b) a peptide comprising extracellular matrix (ECM) protein-binding domain, and
(c) a peptide comprising a domain being recognized by a metalloproteinase,
(d) a peptide comprising a domain for a growth factor,
(e) a peptide comprising a domain being recognized by Lysyl oxidase,
wherein the peptide(s) of one or more of (a) to (e) are preferably covalently attached to the hydrogel.

10. The 3D-scaffold of claim 9, wherein
(a) the peptide of (a) comprises or consists of a linear or cyclic RGD peptide, GFOGERGVEGPOGPA, LPGER, GLPGER, GXOGER, GFOGE, or CDPGYIGSR (SEQ ID NOs 9 to 13),
(b) the peptide of (b) comprises or consists of IQNNKFEYKEDTIKE, TKKTLRT or LRELHLNNN (SEQ ID NOs 14 to 16),
(c) the peptide of (c) comprises or consists of Gly-Leu, Gly-Ile, ALRRLLGLFG, ALRRLLSLF, XXX_{Hy}, L/IXXX_{Hy}, PVPQGS (SEQ ID NOs 17 to 23), wherein X can be any amino acid and X_{Hy} is a hydrophobic amino acid,
(d) the peptide of (d) comprises or consists of MTAASMGPVRVAFVVLLALCSRPAVG, DYLAGFKAHGKKYR and CTCCC (SEQ ID NOs 24 to 26); and/or
(e) the peptide of (e) is rich in Lysyl residues.

11. The 3D-scaffold of any one of claims 1 to 10, wherein the 3D-scaffold comprises viable cells, preferably viable malignant cells, most preferably viable tumor cell or viable cancer cells.

12. A platform for analyzing the viability of cells, wherein the platform comprises at least one unit that comprises
(a) a chamber that comprises one or more, preferably one of the 3D-scaffold of claim 11,
(b) an inlet microchannel that is in fluid connection with the at least one chamber and through which a fluid with a test compound can be introduced into the chamber, and
(c) optionally an outlet microchannel that is in fluid connection with the at least one chamber and through which the fluid with the test compound can leave the chamber.

13. The platform of claim 12 comprising with increasing preference at least 5, at least 10, at least 50 and at least 100 units, preferably in parallel.

14. The platform of claim 12 or 13, wherein
the chamber(s) have a width of about 300-900 µm and a height of about 200-900 µm, and/or
the channel(s) have a width of about 50 µm and a height of about 20-50 µm, and wherein the channel(s) optionally have and inlet or outlet having a width of about 200 µm and a height of about 20 µm.

15. A method for testing whether a test compound or a test condition induces the death, preferably apoptosis of cells comprising
(a) introducing a test compound into the 3D-scaffold of claim 11, or contacting a fluid with a test compound with the 3D-scaffold of claim 11, or contacting a fluid with a test compound with the system of any one of claims 12 to 14, so that the test compound is introduced into the chamber, or applying a test condition to the 3D-scaffold of claim 11, or applying a test condition to the system of any one of claims 12 to 14; and
(b) monitoring the fluorescence of the FRET pair, wherein a decreased or no fluorescence of the FRET acceptor of the FRET pair and/or increased or the onset of fluorescence of the FRET acceptor of the FRET pair upon the contact of the test compound with the cells in the 3D-scaffold(s) or the appliance of the test condition to the cells in the 3D-scaffold(s) indicates that the compound induces the death, preferably apoptosis of the cells.
